# EUROPEAN PATENT APPLICATION

(11) **EP 3 603 688 A1**
(43) Date of publication of application: **05.02.2020**
(21) Application number: 19189454.2
(22) Date of filing: 31.07.2019
(51) Int. Cl.: A61L 29/16, A61L 31/16

(54) **LIMUS COATINGS AND METHODS OF USE THEREOF**

(30) Priority: 31.07.2018 US 201862712629 P
(71) Applicant: Cook Medical Technologies LLC, Bloomington, IN 47404 (US)
(72) Inventor: CERCHIARI, Alec E., Bloomington, IN Indiana 47404 (US); MINDEL, Thomas, Bloomington, IN Indiana 47403 (US); NEIDIGH, Austin, Bloomfield, IN Indiana 47424 (US); BOATMAN, Scott E., Bloomington, IN Indiana 47401 (US); CHAMBERS, Sean D., Bloomington, IN Indiana 47401 (US)
(74) Representative: Arch, Peter Jonathan Sanders

(57) **Abstract**

The present disclosure relates to coatings containing a limus drug and an excipient, to medical devices incorporating such coatings and to methods of preparing and using such devices. In one embodiment, the limus drug is sirolimus. In another embodiment, the excipient is tannic acid or epi gallo catechin gallate

## Description

### TECHNICAL FIELD

The present disclosure generally relates to coatings containing particles of a Limus drug in crystalline form, to medical devices including such coatings and to methods of preparing and using such devices. In one embodiment, the Limus drug is sirolimus.

### BACKGROUND

Local delivery of a therapeutic agent can be useful in the treatment of many medical conditions. Illustratively, local delivery of a therapeutic agent within a body vessel or to a selected portion of internal body tissue can eliminate or reduce the need for systemic delivery of the therapeutic agent thus minimizing any potential adverse effect of the therapeutic agent on areas of the body not needing treatment.

Minimally invasive implantable medical devices, such as balloons, catheters and stents, can provide a platform for delivering therapeutic agents to internal body tissue. For example, balloon catheters or stents may be used to deliver a therapeutic agent directly to the target site within a body vessel such as an artery or vein.

One example of a condition that can be beneficially treated by local administration of a therapeutic agent with a balloon catheter is the delivery of a therapeutic agent in combination with percutaneous transluminal coronary angioplasty (PTCA), a technique used to dilate stenotic portions of blood vessels. Although PTCA and related procedures aid in alleviating intraluminal constrictions, such constrictions or blockages may reoccur in many cases. The cause of these recurring obstructions, termed restenosis, may be due to the body responding to the surgical procedure. Restenosis of the vessel may develop over several months after the procedure, and may require another angioplasty procedure or a surgical bypass operation to correct. Proliferation and migration of smooth muscle cells (SMC) from the media layer of the lumen to the intimal layer cause an excessive production of extracellular matrices (ECM), which is believed to be one of the leading contributors to the development of restenosis. The extensive thickening of tissues narrows the lumen of the blood vessel, constricting or blocking the blood flow through the vessel.

Drugs that inhibit restenosis may be locally delivered during PTCA from a catheter or by placement of a stent configured to continue to release the drug after the PTCA procedure. The delivery of the drug from coatings in these and other minimally invasive procedures can be complicated by the need both to have a coating that is durable during delivery, but which effectively delivers the drug when implanted in the region where local treatment is desired. Because natural biological environments are aqueous, it can occur that a coating containing a water-insoluble drug is sufficiently durable during travel to the intended delivery site, but then fails to optimally deliver the drug at the site. Needs thus exist for compositions, coatings, and coated implantable medical devices which enable the beneficial delivery of a drug locally to a site intended for treatment.

### SUMMARY

One aspect of the present invention relates to a medical device including a base structure having a surface and a coating on the surface comprising a gallate excipient and particles of a Limus drug. In one embodiment at least 70% or 80% of the particles having a maximum dimension greater than 10 micrometers have a maximum dimension less than 25 micrometers. In another embodiment, at least an additional 20% of the particles having a maximum dimension greater than 10 micrometers have a maximum dimension between 25 micrometers and 100 micrometers. In yet another embodiment, less than 0.5 % or 0.2% of the particles have a maximum dimension greater than 10 micrometers have a maximum dimension greater than 100 micrometers.

In other embodiments, the coating is such that, when placed in a physiological environment, at least 70% or 80% of the particles released from the coating that have a maximum dimension greater than 10 micrometers have a maximum dimension less than 25 micrometers. In yet other embodiments, the coating is such that, when placed in a physiological environment, at least a further 20% of the particles released having a maximum dimension greater than 10 micrometers have a maximum dimension between 25 micrometers and 100 micrometers.

In other embodiments, the coating is such that, when placed in a physiological environment, less than 0.5 % or 0.2% of the particles released have a maximum dimension greater than 10 micrometers have a maximum dimension greater than 100 micrometers.

In other embodiments, the excipient is a gallate containing compound, epi gallo catechin gallate, tannic acid or epi catechin gallate.

The medical device may be, for example, a stent, a vascular stent, a ureteral stent, a catheter, a balloon, a balloon catheter, a stent graft, a wire guide or a cannula.

The Limus drug may be present, for example, at a surface density of between 3 and 7 micrograms/mm² on a least a portion of the base surface. The Limus drug may be, for example, sirolimus, pimecrolimus, tacrolimus, everolimus, zotarolimus, novolimus, myolimus, temsirolimus, deforolimus or biolimus.

In some embodiments, the coating containing the drug and, optionally, the excipient, is free of a polymer or non-polymer carrier matrix. In other embodiments, the coating includes less than 1 percentage by weight of a polymer or non-polymer carrier matrix. In yet other embodiments, the excipient is present in an amount effective to increase a rate of release of the drug from the medical device when implanted in a patient.

Another embodiment provides an inflatable balloon including a base structure having a surface and a coating on the surface including a gallate excipient and particles of a Limus drug. When placed in double distilled water at 37C and inflated for 3 minutes, at least 70% of the particles released from the coating having a maximum dimension greater than 10 micrometers have a maximum dimension less than 25 micrometers.

Another aspect of the invention provides a method for preparing a coating on a surface of a medical device. In one embodiment, the method includes preparing a spraying solution including a solvent, a Limus drug and, optionally, an excipient and applying the spraying solution to the surface. In some embodiments, the solvent includes water and a single organic solvent. In some embodiments, the single organic solvent may be a polar organic solvent. In some embodiments, the single organic solvent may be an alcohol. The single organic solvent may be, for example, ethanol. In some embodiments, the solvent contains between 10% and 50% water, or between 20% and 40% water, or between 25% and 35% water, or about 30% water. In some embodiments, the solvent consists of water and the single organic solvent. In some embodiments, the solvent can include water and one or more organic solvents, such as a mixture of organic solvents. In one embodiment, the mixture of organic solvents can include more than one alcohol. In one embodiment, the organic solvents can include some or all of ethanol, methanol, hexane, heptane, dichloromethane, toluene, and benzyl alcohol. For example, one solvent can include water, ethanol, and benzyl alcohol.

The spraying solution may be applied to the surface at a temperature between 70 F and 110 F and/or at a humidity between 20 and 70 RPH.

### BRIEF DESCRIPTION OF THE DRAWINGS

Fig. 1 provides a perspective view of a drug-delivering balloon catheter in accordance with one embodiment of the invention in an inflated condition.
Fig. 2 provides a cross-sectional view of the balloon-mounted region of the balloon catheter of Fig. 1 taken along a central longitudinal axis.
Fig. 3 provides a cross-sectional view of the catheter shaft of the balloon catheter of Fig. 1 taken along line 3-3 and viewed in the direction of the arrows.
Fig. 4 provides a perspective view of the balloon catheter of Fig. 1 in a folded condition.
Fig. 5 provides a cross-sectional view of the balloon catheter of Fig. 4 taken along line 5-5 and viewed in the direction of the arrows.
Fig. 5a provides a cross-sectional view illustrating an alternate coating pattern to that shown in Fig. 5.
Fig. 5b provides a cross-sectional view illustrating another alternate coating pattern to that shown in Fig. 5.
Fig. 6 provides a cross-sectional view of the balloon catheter of Fig. 1 taken along a longitudinal axis and illustrating an alternate coating configuration.
Fig. 7 provides a cross-sectional view of the balloon catheter of Fig. 1 taken along a longitudinal axis and illustrating another alternate coating configuration.
Fig. 8 provides a perspective view of a therapeutic agent-delivering stent in accordance with one embodiment of the invention.
Fig. 9 provides a perspective view of a coated drug-delivering balloon catheter having a coated balloon-expandable stent mounted thereon in accordance with an embodiment of the invention.
Fig. 10 provides a side view of a drug-delivering scoring balloon catheter in accordance with one embodiment of the invention in an inflated condition.
Fig. 11 provides an enlarged cross-sectional view of a dilation element of the scoring balloon catheter of Fig. 10 and adjacent balloon wall film portions.
Fig. 12(A-H) show the effect of variation in drug concentration in the coating solution on the coating properties. Figs. 12(C-G) show macroscopic images of the balloon surface at various sirolimus concentrations. Figs. 12(A) and 12(B) are SEM images of the surfaces coated with differing sirolimus concentrations. Fig. 12(H) is a graph showing the dependency of sirolimus dose density on the surface as a function of sirolimus concentration in the spraying solution.
Fig. 13 (A-I) show the effect of variation in excipient concentration in the coating solution on the coating properties. Figures 13(A-E) show macroscopic images of the balloon surface at various EGCG concentrations. Figs. 13(F-G) are bar charts a graph showing the amounts of sirolimus and EGCG on the surface as a function of the ratio of sirolimus to excipient in the spraying solution. Fig. 13(I) is a graph showing the ratio of drug to excipient as a function of their ratios in the straying solution.
Figure 14 (A) is a graph showing average (n = 6) normalized particulate counts of two sample balloons coated using the methods disclosed herein. Figure 14 (B) depicts the absolute data from the same experiment.

### DETAILED DESCRIPTION OF THE PREFERRED EMBODIMENTS

For the purpose of promoting an understanding of the principles of the invention, reference will now be made to embodiments, some of which are illustrated in the drawings, and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates.

In the discussions that follow, a number of potential features or selections of the drug, excipient, implantable medical device structure, or other aspects, are disclosed. It is to be understood that each such disclosed feature or features can be combined with the generalized features discussed herein, to form a disclosed embodiment of the present invention.

### Definitions

The term "patient" as used herein means a human or veterinary patient.

The term "therapeutic effect" as used herein means an effect which induces, ameliorates or otherwise causes an improvement in the pathological symptoms, disease progression or physiological conditions associated with or resistance to succumbing to a disorder, for example restenosis, of a human or veterinary patient. The term "therapeutically effective amount" as used with respect to a drug means an amount of the drug which imparts a therapeutic effect to the human or veterinary patient.

The term "water-insoluble" as applied to a drug herein refers to a therapeutic agent having a solubility in water at 25°C of less than 1 milligrams per milliliter (mg/mL). More preferably, the water-insoluble drug has a solubility in water at 25°C of less than 0.1 mg/ml, and in certain embodiments less than 10 micrograms per milliliter (µg/ml).

The term "Limus drug" as used herein refers to any therapeutic that inhibits any mammalian target of rapamycin (mTOR) and/or calcineurin.

### Coated Medical Devices

Aspects of the present invention relate to implantable medical devices including a particulate coating including particles of at least one water-insoluble drug at least partially in a crystalline form. In one preferred embodiment, the drug is a Limus drug. In general, Limus drugs are characterized by a triene macrolide lactone ring that hosts a FKBP binding region (i.e. a pipecolate binding site) as well as a mTOR binding region. Typically, drugs in a crystalline solid form dissolve more slowly in an aqueous environment, such as when implanted in the body of a patient, and are retained for longer in the tissue, or in blood, than non-crystalline solid forms. Thus, there are advantages to delivering the drug to the patient in a crystalline form.

However, the crystalline forms are sometimes less durable than the non-crystalline solid forms of a drug. In addition, crystalline forms present in the coatings may include large particulates that, if released into the bloodstream during device delivery or deployment, may present an embolic risk to the patient.

The present embodiments allow for control over the particle size distribution of the Limus drug present in the coating and provide for coatings having a reduced number of large drug particles (for example 200 micrometers) as compared to previously available coatings. The present coatings also provide for control of the particle size distribution of the Limus drug released from the coating in a physiological environment, for example, when an implantable device including the coating is implanted within the body of a patient.

The coatings disclosed herein may also include at least one excipient. As used herein, the term excipient refers to a compound that speeds the release of the drug from the coated device and/or increases the amount of the drug released from the device when the device is implanted in the body of a patient. In certain embodiments, the drug and the excipient are present at a weight ratio of between 10:1 and 1:1; 8:1 and 1:1; 5:1 and 1:1; 4:1 and 1:1; 10:1 and 2:1; 8:1 and 2:1; 5:1 and 2:1; or 10:1 and 1:10 w/w drug to excipient. The excipient may be, for example, a gallate containing compound such as, but not limited to, epi gallo catechin gallate (EGCG), tannic acid or epi catechin gallate.

In one embodiment, the Limus drug is present in a form such that at least 70 percentage of particles of the drug in the coating, or that are released from the coating, having a maximum dimension greater than 10 micrometers have a maximum dimension less than 25 micrometers. Optionally, at least a further 20 percentage of these particles have a maximum dimension between 25 micrometers and 100 micrometers. Optionally, less than 0.5 percentage or 0.2 percentage of 0.1 percentage of these particles have a maximum dimension greater than 100 micrometers. In this embodiment and the embodiments disclosed below, the size of particles released from the coating is determined as follows.

Drug coated balloons (DCBs) are fully submerged in a glass test tube containing at least 25 mL of distilled deionized water (ddH20) at 37° C. The DCBs are then inflated at nominal pressure (e.g. 8 atm for drug coated Advance® 14LP angioplasty balloons) for 3 minutes using saline solution. During inflation, each balloon remains fully submerged in the test tube. Following inflation, balloons are deflated and extracted from the test tube. A magnetic stir bar is then added to each test tube and allowed to gently stir the solution at 460 rpm. While stirring, the ddH20 is sampled three times and analyzed using a Liquid Particle counter (e.g. Hiac Royco, Beckman Coulter Life Sciences, Indiana 46268, USA). Finally, the size and total number of particles that detached from the balloon is quantified. This method is also applicable to determine the particle size distribution of drug released from other devices, including non-inflatable devices.

In yet another embodiment, the Limus drug is present in a form such that at least 80 percentage of particles of the drug in the coating or released from the coating and having a maximum dimension greater than 10 micrometers, have a maximum dimension less than 25 micrometers. Optionally, at least a further 15 percentage of the particles have a maximum dimension between 25 micrometers and 100 micrometers. Optionally, less than 0.5 percentage or 0.2 percentage of 0.1 percentage of these particles present in, or released from, the coating have a maximum dimension greater than 100 micrometers.

In yet another embodiment, the Limus drug is present in a form such that at least 85 percentage of particles of the drug in the coating or released from the coating having a maximum dimension greater than 10 micrometers have a maximum dimension less than 25 micrometers. Optionally, at least a further 10 percentage of the particles have a maximum dimension between 25 micrometers and 100 micrometers. Optionally, less than 0.5 percentage or 0.2 percentage of 0.1 percentage of the particles have a maximum dimension greater than 100 micrometers.

In another embodiment, the Limus drug is present in a form such that at least 90 percentage of particles of the drug in the coating or released from the coating having a maximum dimension greater than 10 micrometers have a maximum dimension less than 25 micrometers. Optionally, at least a further 5 percentage of the particles have a maximum dimension between 25 micrometers and 100 micrometers. Optionally, less than 0.5 percentage or 0.2 percentage of 0.1 percentage of these particles have a maximum dimension greater than 100 micrometers.

In yet another embodiment, the Limus drug is present in a form such that at least 95 percentage of particles of the drug in the coating or released from the coating having a maximum dimension greater than 10 micrometers have a maximum dimension less than 25 micrometers. Optionally, at least a further 4 percentage of the particles have a maximum dimension between 25 micrometers and 100 micrometers. Optionally, less than 0.5 percentage or 0.2 percentage of 0.1 percentage of these particles may have a maximum dimension greater than 100 micrometers.

Limus drugs having at least one crystalline form that are within the scope of the present embodiments include a macrolide immunosuppressive agent such as sirolimus (rapamycin), pimecrolimus, tacrolimus, everolimus, zotarolimus, novolimus, myolimus, temsirolimus, deforolimus, or biolimus; an antiproliferative agent; a smooth muscle cell inhibitor; an inhibitor of the mammalian target of rapamycin (mTOR inhibitor); or a mixture of two, or two or more of any of these. These or other water-insoluble restenosis-inhibiting agents, including each agent or agent type identified herein, more preferably have a solubility in water at 25 C of less than 1 mg/ml, even more preferably less than 0.1 mg/ml, and in certain embodiments less than 10 micrograms/ml. Sirolimus, pimecrolimus, tacrolimus, everolimus, zotarolimus, novolimus, myolimus, temsirolimus, deforolimus, and biolimus are preferred water-insoluble restenosis-inhibiting agents for use herein (each known to have a water solubility of less than about 10 micrograms/ml). In one particularly preferred embodiment the drug is sirolimus.

The medical device may be any of a wide variety of devices having an implantable medical device structure sized and shaped for temporary or permanent implantation in a patient. Medical devices having structures implantable in a bodily passage will often be used. The bodily passage may for example be a passage of the alimentary system, the urogenital system, the biliary system, or the cardiovascular system. Medical devices including a device structure implantable in the cardiovascular system are preferred, including for example those implantable in a vessel or chamber of the cardiovascular system of a human or animal patient through which blood travels. The passage may for example be a tubular passage such as an artery or vein, or may be a larger chamber such as a ventricle or atrium of the heart. Implantable medical devices that include structures that span or bridge between cardiovascular or other bodily passages are also contemplated. The implantable medical device can be adapted to be entirely or only partially implanted in a cardiovascular passage or other bodily passage.

By way of example, the medical device can be or include a catheter, a wire guide, a stent, a coil, a needle, a graft, a filter, a balloon, a cutting balloon, a scoring balloon, a weeping (perfusion) balloon, or any combination of these. Preferred devices include, a stent, a vascular stent, a ureteral stent, a catheter, a balloon, a balloon catheter, a stent graft, a wire guide and a cannula.

Suitable filters include for example vena cava filters such as the Cook CELECT® and Cook Günther TULIP® and Cook Gianturco-Roehm Bird's NEST® filters available from Cook Medical, Bloomington Indiana, USA. Suitable stents include those without a covering, for example the Cook ZILVER® stents available from Cook Medical. Suitable stents also include those with a sheath covering. Suitable coils include embolization coils. Suitable wire guides include for instance traditional wire guides as well as wire guides with an attached expandable structure for expansion within a blood vessel lumen, such as a coil, where the expandable structure can optionally carry the coating or coatings as disclosed herein. These or other implants, in certain preferred embodiments, have at least a portion that is configured to expand during deployment so as to contact walls of the passage in which they are implanted to anchor within the passage.

The implantable medical device can be made from any suitable material or combination of materials. Illustratively, the implantable medical device can include a metal such as stainless steel, tantalum, titanium, nitinol, cobalt, chromium, nickel, molybdenum, manganese, gold, platinum, inconel, iridium, silver, tungsten, elgiloy, alloys of any of these, or another biocompatible metal; carbon or carbon fiber; a calcium-containing inorganic material such as a ceramic; a material composed of ceramic and metallic components (cermet); or a polymeric material. The material of construction for the implantable medical device structure can be biodegradable or non-biodegradable.

Nonbiodegradable polymers that can be used include, for example, cellulose acetate, cellulose nitrate, silicone, polyethylene terephthalate, polyurethane, polyamide, polyester (e.g. Nylon), polyorthoester, polyanhydride, polyether sulfone, polycarbonate, polypropylene, high molecular weight polyethylene, and polytetrafluoroethylene, or mixtures of these. Biodegradable polymers that can be used include, for example, polylactic acid (PLA), polyglycolic acid (PGA), poly(lactic-co-glycolic acid) (PLGA), polyanhydride, polycaprolactone, polyhydroxybutyrate valerate, or mixtures of these. Biodegradable metals may also be used, including for example a biodegradable magnesium alloy.

In some preferred embodiments herein, the implantable medical device will be or include a balloon catheter, such as an angioplasty balloon catheter, a weeping or infusion balloon, a scoring balloon catheter or a cutting balloon catheter. Such a balloon catheter can include at least one balloon mounted on a catheter shaft, with the catheter shaft defining an inflation lumen fluidly communicating with an interior of the balloon. The catheter shaft can also define a guide member lumen, for receiving an elongate guidewire or other guiding member for the catheter. The guide member lumen can extend from a distal opening distal to the balloon to a proximal opening proximal to the balloon. The proximal guide member lumen opening can occur in a sidewall of the catheter shaft in a region proximate to the balloon (e.g. within about 10 cm proximal to the proximal end of the balloon) and which is positioned to reside within the patient during use of the balloon catheter, as occurs for example in "rapid-exchange" balloon catheter constructions, or can occur on the catheter shaft in a region positioned to reside external of the patient during use of the balloon catheter, as occurs for example in so-called "over-the-wire" balloon catheter constructions. The balloon catheter may include multiple balloons, usually in this case only two balloons, mounted in positions spaced longitudinally from one another on the catheter shaft. In such cases the balloons may share a common inflation lumen defined by the catheter shaft, or each may have a separate inflation lumen defined by the catheter shaft. In such balloon catheters having only two, or two or more balloons, the distal opening of the guide member lumen can occur distally of the distal-most balloon, and the proximal opening of the guide member lumen can occur proximal of the proximal-most balloon, in either rapid-exchange or over-the-wire type configurations as discussed above.

The balloon(s) of the balloon catheters herein may be configured for vascular angioplasty, and/or may have a balloon wall made of any suitable balloon wall material, typically a polymeric balloon wall material. The polymeric or other balloon wall material can be elastomeric, as in the case of an illustrative silicone elastomer, latex rubber elastomer, nylon elastomer, or polyurethane elastomer balloon film, where the balloon can expand upon inflation due to the expansion and thinning of the balloon wall material. The compliance of the balloon wall material in such elastomeric balloon applications is typically greater than 20% and more typically greater than 50%, and/or the burst pressure of such elastomeric balloons will typically be in the range of about 1.1 to about 2 atmospheres. In other embodiments, the polymeric or other balloon wall material can be inelastic, as in the case of a non-compliant or semi-compliant balloon (e.g. as commonly used in angioplasty and/or stent delivery balloons), where the balloon can expand upon inflation due to the unfolding of the balloon wall material from an initial folded configuration. Preferred balloon wall materials for non-compliant or semi-compliant balloons include polyamide (e.g. as in Nylon balloons), polyethylene terephthalate (PET), or polyurethane polymers. At least a portion of and potentially the entirety of such an elongate, generally cylindrical outer surface can carry the drug and excipient as discussed herein, either as the sole coating carried by the generally cylindrical outer surface or in combination with one or more additional coatings carried by the generally cylindrical outer surface.

In other preferred embodiments herein, the implantable medical device will be or include a stent. Such a stent may for example be a force-expandable stent, such as a balloon-expandable stent, or a self-expanding stent. The stent may be made from any one of numerous metals and alloys, including those identified hereinabove. The structure of the stent may be formed in a variety of ways to provide a suitable intraluminal support structure having an outer surface for contact with the vessel wall upon implantation and an inner surface that faces the lumen of the vessel and that can be generally opposite the outer surface. For example, the stent may be made from a woven wire structure, a laser-cut cannula, individual interconnected rings, or another pattern or design. In these or other constructions, the stent can include a plurality of struts each having an outer surface for contact with the vessel wall and an inner surface for facing the lumen of the vessel.

Such stents may be force-expandable, such as balloon-expandable, or self-expanding, as discussed above. Self-expanding stents of this type can be made of a resilient metal, preferably a superelastic metal alloy such as a superelastic nickel-titanium (Ni-Ti) alloy, as occurs for example in the ZILVER® NITINOL stent commercially available from Cook Medical.

Any stent discussed above or elsewhere herein can have a stent surface carrying the coating as discussed herein, either as the sole coating carried by the stent surface, or in combination with one or more additional coatings positioned underneath and/or overtop the layer containing the drug. As well, surfaces of the stent not carrying the drug may optionally be bare (uncoated), or may carry one or more different coatings. Additionally, where the stent is mounted on a balloon of a balloon catheter for delivery, the surface of the balloon may carry the drug and potentially other layer(s) as described herein, and/or the surface of the stent may carry the drug and potentially other layer(s) as described herein. The practice of these and other variants will be within the purview of those of ordinary skill in the art in view of the teachings herein.

In some embodiments, the Limus drug and excipient (if present) constitute greater than 50, 75, 90, 95, 99 or 100 percentage by weight of the coating. In other embodiments, the coatings include less than about 5, 2, 1, 0.5, 0.1, 0.05 or 0.01 percentage by weight of materials other than the drug and the excipient (if present). In yet other embodiments, the coatings include less than about 5, 2, 1, 0.5, 0.1, 0.05 or 0.01 percentage by weight of other materials, such as polymer or non-polymer carriers, that alter the release rate of the drug from the device when implanted. In yet other embodiments, only the drug and the excipient (if present) are present in the coating.

The drug can be incorporated in the device at any suitable level. Typically, when coated onto a device such as a stent or a balloon, the drug will be incorporated at a level of 0.1 to 1000 micrograms per mm², or 0.1 to 100 micrograms per mm², and in certain preferred forms 0.1 to 10 micrograms per mm², or 3 to 6 micrograms per mm², or 0.5 to about 2 micrograms per mm² of the coated surface. Where two or more drugs are included in the coating, the above-recited levels can apply to the combined weight of all the drug(s), or to the drug(s) individually.

It will also be understood that the coating may contain variations in the level of drug in different regions of the coating either due to manufacturing variances or intentional design criteria. Thus, the present invention contemplates coatings in which the level of drug(s) is substantially uniform over the entire area covered by the coating, or in which the level of drug(s) differs substantially in one area of the coating as compared to another area covered by another area of the coating.

In certain preferred embodiments, a Limus drug (for example sirolimus) is incorporated at a level in the range of 1 microgram per mm² to 10 micrograms per mm², or in the range of 3 micrograms per mm² to 6 micrograms per mm², or in the range of 3 micrograms per mm² to 7 micrograms per mm², or in the range of 0.5 micrograms per mm² to 2 micrograms per mm² either as the only drug in the coating or in combination with one or more additional drugs.

In particularly beneficial implantable medical devices of the invention, such Limus drug-containing coatings are carried on a surface of a stent, including for example any stent described herein, and/or on a surface of a balloon of a balloon catheter, including for example any balloon catheter described herein.

The drug will typically be incorporated in the device in a therapeutically effective amount. In this regard, it will be understood that where the drug is a restenosis-inhibiting agent, the restonosis-inhibiting agent will be incorporated in the coating in an amount that is effective to inhibit restenosis when the implantable medical device (e.g. a balloon or stent) is deployed so as to deliver the drug from the implantable medical device to a wall of the artery, vein or other vessel or passage that is being treated by the device. As will be recognized, the level of a drug that will be therapeutically effective will vary in accordance with the particular drug in use, the implantable medical device in use, the implant site, the condition to be treated, the composition of the coating including the drug, and other potential factors. Through routine experimentation in view of the disclosures herein the achievement of a therapeutically effective amount of drug will be within the purview of those of ordinary skilled in the field.

The excipient can be included in the device in an amount effective to increase the rate of release of the drug from the device at a site of implant of the implantable medical device structure (as compared to the rate of release from an otherwise identical device not including the excipient). In other embodiments, the excipient is present in an amount that increases the amount of drug released from the device as compared to an otherwise identical device not including the excipient. This capacity can be demonstrated in, for example, *in vivo* testing, or in vitro testing where the level of the excipient are observed to increase the rate of release of the drug(s) in water, or in an aqueous medium such as blood serum or a 0.2 weight % aqueous solution of Heptakis (2,6-O-methyl)-beta-cyclodextrin (HCD), under static conditions at a temperature of 37°C.

In embodiments in which the drug is contained in a layer coating the implantable medical device, the excipient may increase the amount of drug released when implanted by 10, 20, 30, 40, 50 75, 100, 125, 150, 200, 300 or 400 percentage as compared to a device that is identical except for the absence of the excipient.

In certain other embodiments, the excipient is observed to increase the delivery of the drug across a vessel wall and into the tissue of the patient. The increase in the amount of the drug delivered to the tissue of the patient from a device including the excipient may depend upon a number of factors, such as the nature of the vessel in which the composition is placed or device is implanted, as well as the environment within the vessel and the construction of the implantable device.

However, the increase in the amount drug delivered through a vessel wall may be characterized in an *ex vivo* assay in which the implantable device is placed in a section of the appropriate vessel and incubated in a buffer solution for a fixed time. In one such assay, a device is placed in a section of porcine ureter, which is hydrated in a Phosphate Buffered Saline buffer. The ureter is incubated in a closed container for a fixed time, for example 5 minutes at 37 deg. C. After the incubation period, the drug present in the ureter tissue is extracted using an extraction solution, such as an enzyme solution, organic solvent, organic/aqueous mixture, or acidified mixture. The extraction solution used is dependent on the drug being extracted. The amount of drug present in the ureter and in the device is determined using, for example, an appropriate HPLC method.

In certain embodiments, the claimed devices include excipient sufficient to increase the amount of drug delivered, as measured by this assay, by at least 5%, 10%, 15%, 20%, 25%, 30%, 40%, 50%, 70%, 100%, 150% or 200% compared to the amount of drug delivered, under the same conditions, from an otherwise identical device that does not include the excipient. In other embodiments, the devices include excipient sufficient to increase the amount of drug delivered, as measured by this assay, within a period of 1, 5, 15, 30, 60, 120, 300, 500 or 1000 minutes by at least 5%, 10%, 15%, 20%, 25%, 30%, 40%, 50%, 70%, 100%, 150% or 200%. In yet other embodiments, the claimed devices include an amount of excipient sufficient to increase the amount of drug delivered, as measured by this assay, within a period of 1, 5, 15, 30, 60 or 120 days by at least 5%, 10%, 15%, 20%, 25%, 30%, 40%, 50%, 70%, 100%, 150% or 200%.

In certain embodiments, the excipient is effective to deliver a therapeutically effective amount of the drug to patient tissue in a time period of about 5 minutes or less after implantation of the implantable medical device. More preferably, such time period is about 3 minutes or less, even more preferably about 2 minutes or less, and most preferably about 1 minute or less, e.g. in the range of about 20 seconds to about 1 minute. Such embodiments configured for relatively rapid delivery are especially beneficial when the drug is carried by a surface of a temporarily implantable medical device structure, for example a balloon of a balloon catheter, including any balloon catheter and in any coating arrangement described herein.

In other embodiments, the excipient increases the durability of the coating to resist the undesirable premature release of the drug from a device prior to delivery to a point of treatment within a body vessel. For example, the excipient can decrease the amount of drug released prematurely during folding and unfolding of a balloon portion of a balloon catheter or during crimping of an expandable stent. Likewise, the excipient may decrease the amount of drug loss during delivery of a medical device, such as a balloon or stent, to the treatment site.

In certain embodiments, coated stents disclosed herein exhibit a drug amount loss of less than about 10%, more preferably less than about 8%, 6%, 4%, 3%, 2%, 1% or 0.5% and most preferably less than about 0.1% upon crimping to a diameter of 6 French (6F) or 4 French (4F) and expansion. In other embodiments, coated balloons disclosed herein exhibit a drug amount loss of less than about 10%, more preferably less than about 8%, 6%, 4%, 3%, 2%, 1% or 0.5% and most preferably less than about 0.1% during folding and unfolding of the balloon.

A wide variety of coating patterns may be used to coat the medical device. The coating layer can be directly adhered to a surface of an implantable structure of the medical device and provide an outermost surface over the implantable structure, and/or to constitute the entirety of the overall material coat on the implantable structure. In other embodiments, an overall material coat adhered to the implantable structure of the medical device can include one or more different coatings positioned underneath the layer including the drug (e.g. as in a polymeric or other primer coating, or a different drug coating, adhered directly to the surface of the medical device), one or more different coatings positioned overtop the layer including the drug (e.g. as in a polymeric or other protective or diffusion barrier coating), or both. As well, there may be one or more different coatings adjacent the layer including the drug, and/or multiple layers including the drug may be carried by the implantable medical device at locations discrete from one another. The layer including the drug may be present in an aperture(s) such as a well(s), groove(s) or hole(s) defined in the implantable medical device (e.g. in a stent) or may partially coat or completely coat the implantable medical device or a given surface (e.g. inner, outer or side surface) of the implantable medical device. These and other overall device coating arrangements can be utilized.

The layer including the drug can be carried by any suitable surface of the implantable medical device structure. The layer including the drug can be carried by, and in some embodiments only by, a surface or surfaces of the implantable medical device configured for contact with patient tissue when the device is implanted. For example, in some embodiments the layer including the drug is carried by a surface of a balloon of a balloon catheter, or by a surface of a stent, which is configured for contact with a wall of a vessel when the balloon is implanted (usually temporarily) or when the stent is implanted (usually permanently). In particular embodiments, in the case of a balloon of a balloon catheter which inflates to provide a substantially cylindrical outer surface as discussed above, the layer including the drug is carried by such substantially cylindrical outer surface, either partially or completely covering the substantially cylindrical surface. In the case of a stent having an outer surface as discussed above, the layer including the drug can be carried by the outer surface, either partially or completely covering the outer surface.

### Methods of Coating

The layer including the Limus drug and any other coating layers present can be incorporated as a part of the implantable medical device by any suitable method. This layer and any other coating layer can be formed on a surface of the implantable medical device. For example, the layer or other coating layer(s) can be formed by a method that includes dipping, spraying, showering, dripping, or otherwise applying a medium containing the coating ingredients, and optionally a substance such as a solvent can be removed from the medium to leave the coating adhered to the implantable medical device.

Spray coating is one preferred form of applying the coating materials to the surface of the implantable medical device, and in particular embodiments ultrasonic spray coating will be utilized. During spray coating or other coating operations, the implantable medical device can be moved relative to a sprayer or other applicator of the coating ingredients. This can occur by moving the implantable medical device (including for example rotating the device or at least the portion to be coated), moving the sprayer or other applicator, or both. Multiple application passes or steps will typically be utilized to increase the thickness of the layer including the drug or other coating layer(s) and control the levels of the drug, excipient, or other ingredients applied to the implantable medical device. In spray or other application processes, areas of the implantable medical device adjacent to areas desired for coating can optionally be masked to prevent the application of coating materials to the masked areas, and /or portions of applied coating materials can be removed to selectively leave a layer including the drug or other coating in a desired region or regions of the device.

The layer including the drug can be constituted entirely of the drug, the drug and excipient, or may, for example, include a biostable polymer, where the polymer remains attached to the device structure as the drug is released. Alternatively, or in addition to the biostable polymer, this layer may include a bioabsorbable polymer. Such a polymer layer can include a polymeric matrix, for example made using a suitable polymer as identified herein, and in certain forms will be a porous layer that releasably contains an admixture including the drug and excipient in the pores thereof.

Preferably, the coating is applied using a spray coating method such as is disclosed in detail in the examples below. In preferred embodiments the drug (for example sirolimus) and excipient (for example, Tannic acid or EGCG) are dissolved in a solvent and applied to the surface using a spray gun. In preferred embodiments, the solvent includes water and a single organic solvent, for example ethanol. Other organic solvents suitable for the preparation of the coatings include methyl pyrrolidinone, butyrolactone, dimethyl isosorbide, tryethylene glycol dimethyl ether, ethoxy diglycol, glycerol formal, dimethyl formamide, dimethyl acetamide, dimethyl sulfoxide or benzyl alcohol. In other embodiments, the solvent includes only water and a single organic solvent.

We have discovered that control of the particle size distribution and crystalline form of the drug in the coating may be obtained by, for example, variation in the amount of water and/or excipient in the spray solvent; variation of the fluid pressure and atomization pressure used for spraying; or variation of environmental humidity and temperature. For instance, we have discovered that the portion of crystalline drug may be controlled by varying the amount of water in the solvent. Inclusion of water in the coating solvent in such relatively high quantities, rather than an organic solvent component, yields unexpectedly good results, particularly in view of the fact that large amounts of water can cause the drug to precipitate. Preferably the solvent contains at least 30%, 35% or 40% w/w water, with the organic solvent making up the remainder of the solvent. In other embodiments, the solvent contains between 20% and 30%, or 20% and 40%, or 20% and 50%, or 30% and 40%, or 30% and 50%, or 40% and 50% water. Particle size may also be controlled by varying the amount of excipient in the solvent.

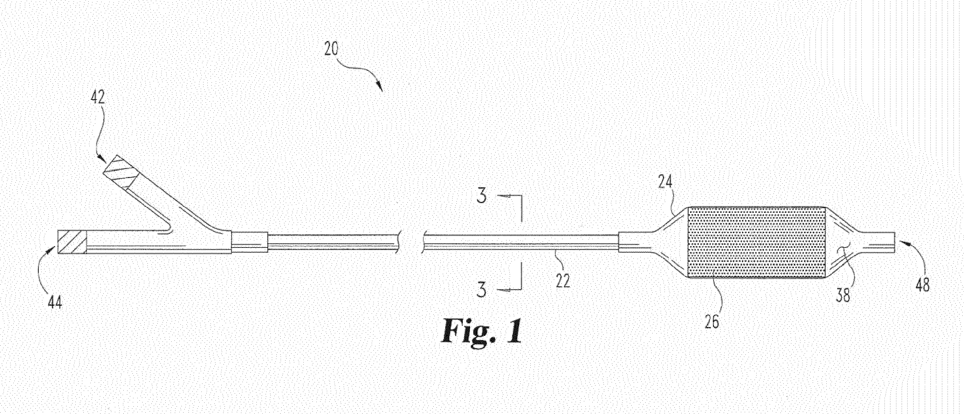

With reference now to Figs. 1-5, shown is one embodiment of a drug-delivering balloon catheter 20 in accordance with the embodiments of the invention. Balloon catheter 20 includes a catheter shaft 22 and a balloon 24 mounted thereon. A material coat 26 including a layer containing a drug and excipient 26a as described herein is carried by balloon 24. Catheter shaft 22 includes a first lumen 28 and second lumen 30. Lumen 28 is configured for inflation of balloon 24, and lumen 30 is configured to receive a guide wire 32 or other guide member to be used in conjunction with balloon catheter 20. Balloon 24 includes an interior region 34 designed to receive a liquid or other fluid for inflation of balloon 24. Balloon 24 has an inner wall 36 bounding balloon interior 34, and an outer wall surface 38. Layer 26 is adhered to outer wall surface 38 of balloon 24.

Balloon catheter 20 also includes a catheter hub 40 mounted to shaft 22. Catheter hub 40 defines a first opening 42 which fluidly communicates with balloon inflation lumen 28, and a second opening 44 which fluidly communicates with lumen 30 defined by shaft 22. Opening 42 of hub 40 and lumen 28 communicate with an opening 46 into the interior 34 of balloon 24, for passage of the inflation fluid for the balloon 24. Opening 44 of hub 40 and lumen 30 defined by a catheter shaft 22 extend to distal opening 48 of lumen 30, with distal opening 48 positioned distally of balloon 24.

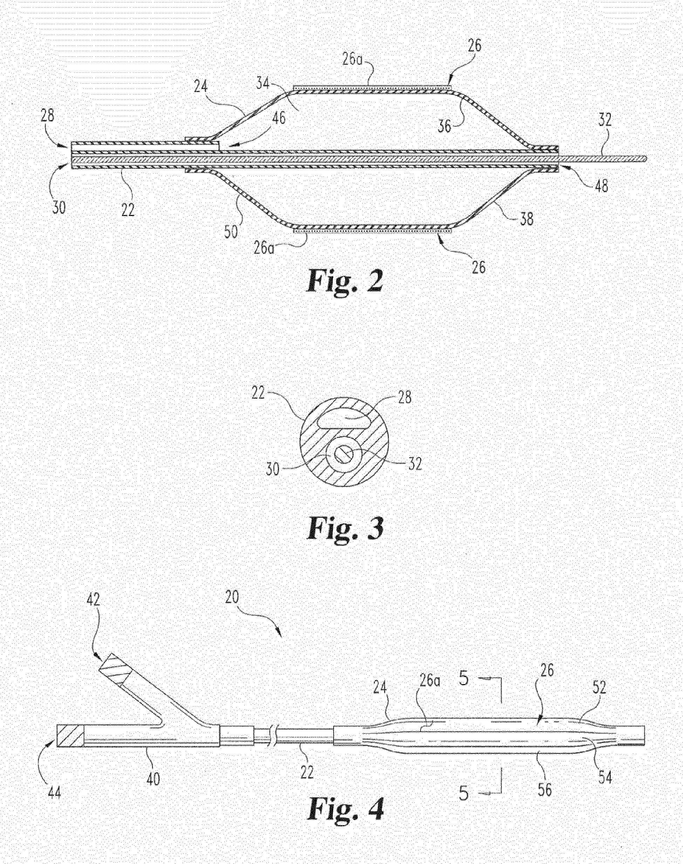

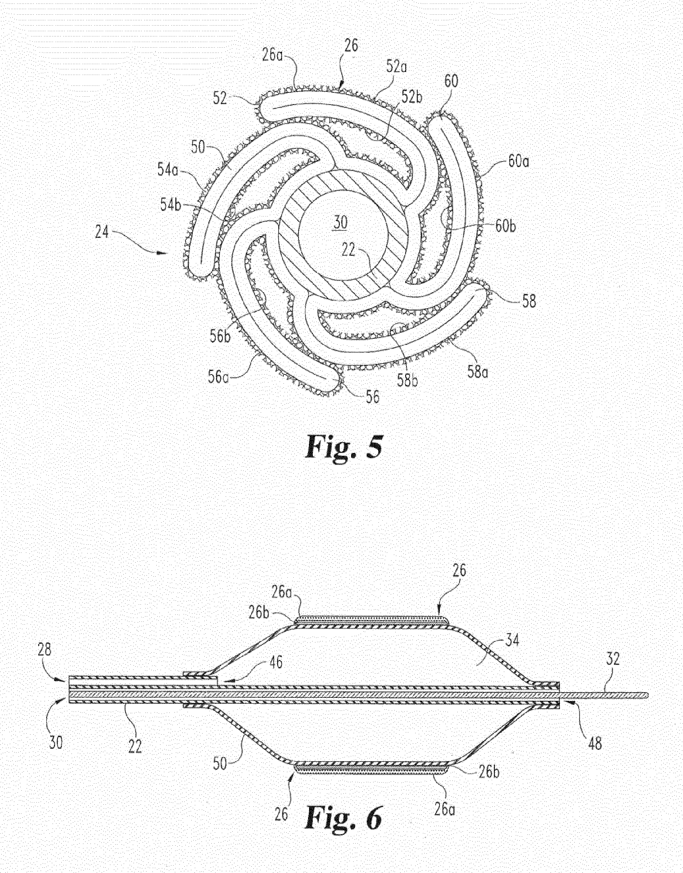

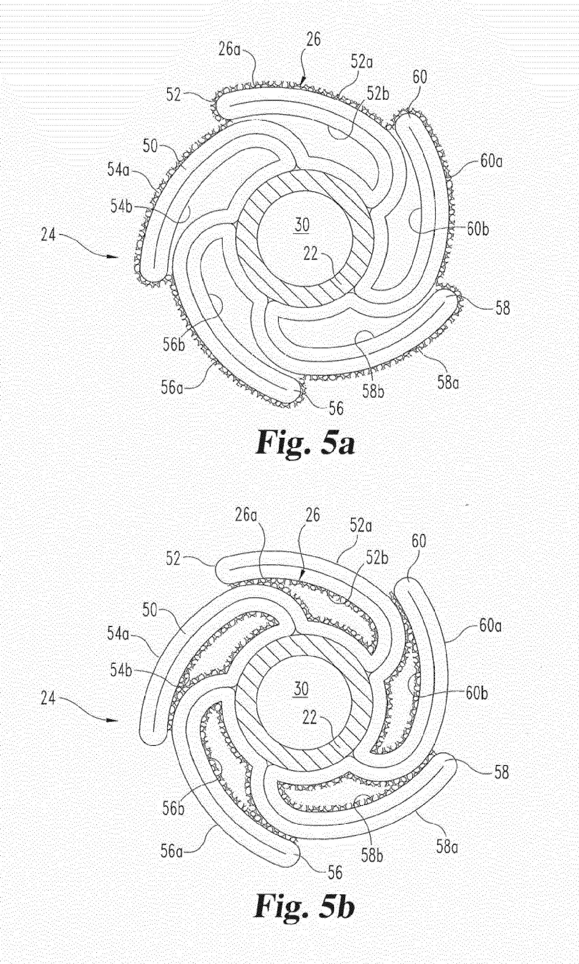

With reference to Fig. 5 still in conjunction with features shown in Figs. 1-4, balloon 24 includes a balloon wall 50, for example defined by a conventional balloon film, typically made from a polymeric material such as one of those discussed hereinabove. Balloon wall 50 as shown in Figs. 4 and 5 is in a folded condition, useful during insertion of balloon 24 into a vessel such as an artery or vein. In its folded condition, balloon 24 includes pleats 52, 54, 56, 58, and 60. As shown, pleats 52-60 are arranged in a spiral pattern with each pleat in a curved condition extending circumferentially around the portion of catheter shaft 22 over which they occur, with the pleats overlapping and thereby contacting one another along at least a portion of their length. In this folded arrangement, pleats 52, 54, 56, 58, and 60 include externally-exposed pleat surfaces 52a, 54a, 56a, 58a, and 60a, and internal non-exposed pleat surfaces 52b, 54b, 56b, 58b, and 60b. Correspondingly, material coat 26, which in the illustrated embodiment includes coating layer 26a, has externally-exposed portions positioned on externally-exposed pleat surfaces 52a-60a, and internal non-exposed portions positioned on internal non-exposed pleat surfaces 52b-60b. Also in this arrangement, because pleats 52-60 overlap with one another, regions of material coat 26 and its drug and excipient 26a are in contact with other regions of material coat 26 and its drug and excipient 26a. In reference to Fig. 5, it should be understood that the features shown therein are intended to be illustrative, and that in practice balloon 24 is typically tightly pleated and wrapped around catheter shaft 22 and thus there will often be little or no open space on the interior of pleats 52-60.

Referring now to Fig. 6, shown is another embodiment of a balloon catheter having features similar to those of balloon catheter 20 of Figs. 1-5, but wherein material coat 26 includes a first coating layer 26a, which is a layer containing drug and excipient as described herein, and a second coating layer 26b different from this layer and positioned underneath coating layer 26a. Coating layer 26b may, in certain embodiments, be a polymeric primer layer as discussed above.

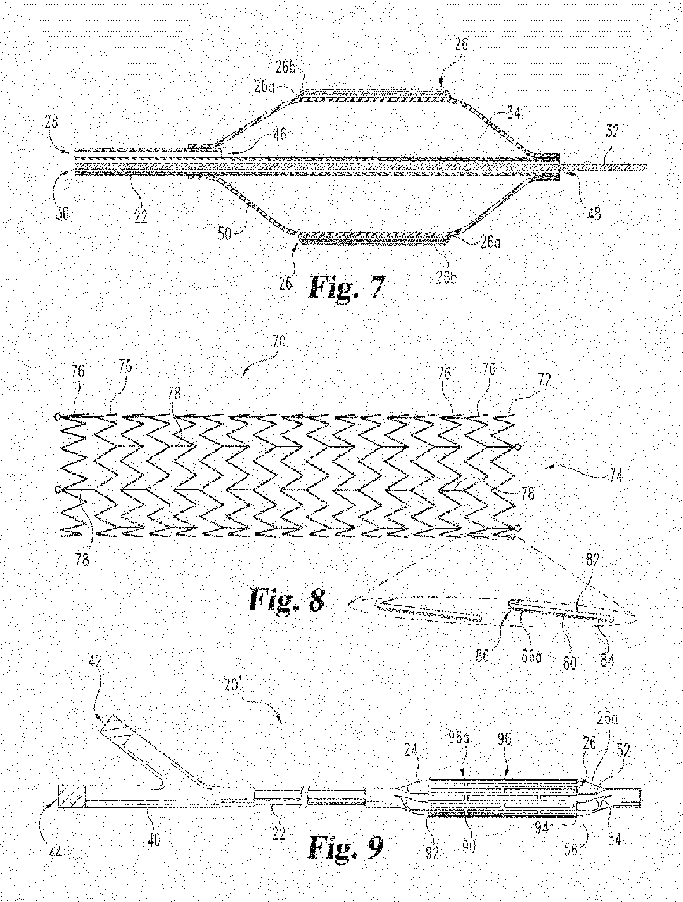

Referring to Fig. 7, shown is another embodiment of a balloon catheter similar to balloon catheter 20 of Figs. 1-5, except wherein material coat 26 includes a first coating layer 26a, which is a layer containing drug and excipient as described herein, adhered directly to the outer surface 38 of balloon 24, and a second coating layer 26b positioned overtop coating layer 26a. Coating layer 26b of Fig. 7 may, in certain embodiments, be a polymeric protective layer and/or a polymeric diffusion barrier layer operable to control the release of the drug through the diffusion barrier layer.

Figs. 5a and 5b show balloon catheter embodiments similar to that shown in Figs. 1-5 except having a different coating pattern for material coat 26. In particular, in Fig. 5a, the material coat 26 including the layer containing drug and excipient 26a is carried only by the externally exposed surfaces 52a-60a of pleats 52-60. This configuration may be prepared, for example, by coating selected surface areas of the balloon 24 while in the inflated condition that will upon folding be positioned as externally exposed pleat surfaces 52a-60a, or by coating balloon 24 while in the folded condition under folding and coating conditions that coat only the externally exposed pleat surfaces 52a-60a. Fig. 5b discloses a balloon catheter embodiment in which the material coat 26 is carried only by internal non-exposed pleat surfaces 52b-60b. This configuration may be prepared, for example, by coating selected surface areas of the balloon 24 while in the inflated condition that will upon folding be positioned as internal non-exposed pleat surfaces 52b-60b, or by coating balloon 24 completely circumferentially while in the inflated condition, pleating and folding the balloon, and then removing the material coat 26 portions on the externally exposed pleat surfaces 52a-60a, for example mechanically and/or with a solvent or other medium capable of displacing the material coat 26. Layer 26a of the embodiments of Figs. 5a and 5b can be applied in any suitable fashion, including using any of those methods described herein. As well, the material coat 26 of the embodiments of Figs. 5a and 5b can, in other embodiments, be a multi-layer coating such as those shown and described herein, including those shown and described in conjunction with Figs. 6 and 7.

Fig. 8 illustrates another embodiment of the invention. A stent 70 includes a stent body 72 defining a central lumen 74. Stent body 72 includes a plurality of longitudinally-adjacent segments 76 including struts defining a circumferential path about lumen 74 and a pattern of connecting strut segments 78 connecting adjacent segments 76. A coating 86 (see exploded section, lower right) including a layer containing drug and excipient 86a is carried by a surface of stent 70. In the illustrated embodiment, the layer 86a is adhered directly on the surface of stent 72 as the sole coating, although in other embodiments the stent coating 86 may be a multi-layer coating such as those shown and described herein, including those coatings shown and described in conjunction with Figs. 6 and 7. Stent 72 has outer strut surfaces 80 configured for contact with a vessel wall such as an artery or vein wall of a patient. Stent 72 has an inner strut surface 82 opposite the outer surfaces 80 and generally facing the lumen 74. Stent 72 also has strut sidewall surfaces 84 between outer and inner strut surfaces 80 and 82. Strut outer surfaces 80 carry the material coat 86 over at least a portion of the outer surface of stent 72 and in certain embodiments over the entire or essentially the entire outer surface of stent 72. Stent 72 is desirably a self-expanding stent and is preferably made of a resilient metal, preferably a superelastic metal alloy such as a superelastic nickel-titanium (Ni-Ti) alloy, as occurs for example in the ZILVER® nitinol stent commercially available from Cook Medical, Bloomington, Indiana, USA. Stent 72 can be manufactured using methods and materials disclosed herein for stents or otherwise, and the layer 86a and any other coating(s) present on the stent may have any composition taught herein and may be incorporated onto stent 72 in any suitable fashion, including any of those disclosed herein.

Fig. 9 illustrates another embodiment of the invention. The implantable medical device 20' of Fig. 9 is similar to that shown in Fig. 4, except also having a balloon-expandable stent 90 mounted over balloon 24. Stent 90 has a proximal end 92 and a distal end 94. In this or other balloon catheters having a stent mounted on the balloon, either stent 90, balloon 24, or both, can carry a layer containing drug and excipient on a surface thereof. In the illustrated embodiment 20', the stent 90 has a material coat 96 including a layer containing drug and excipient 96a carried by an external surface thereof, and the balloon 24 has a material coat 26 including a layer containing drug and excipient 26a carried by the surface of balloon 24. The coating layer 26a can be carried on the balloon 24 so as to extend proximally of proximal end 92 of stent 90 and distally of distal end 94 of stent 90. In this fashion, the drug(s) of the layer containing drug and excipient, when balloon 24 is inflated in a vessel such as an artery or vein to dilate the vessel and implant the stent 90, can be applied to the vessel in regions extending proximally and distally of the stent 90. Where the drug(s) is or includes a restenosis-inhibiting agent, this can inhibit restenosis that may otherwise occur due to edge effects experienced at or near the proximal 92 and distal 94 ends of the stent 90.

The balloon and other components of the balloon catheter of device 20', and the stent 90, can be manufactured using methods and materials disclosed herein for the same or otherwise. The material coat 26 and/or material coat 96 can include a sole layer 26a or 96a adhered directly to the surface of balloon 24 or stent 90, respectively, although in other embodiments the balloon material coat 26 or stent material coat 96 may be a multi-layer coating such as those shown and described herein, including those coatings shown and described in conjunction with Figs. 6 and 7. The layer containing drug and excipient and any other coating layer(s) present on the balloon and/or stent of device 20' may have any composition taught herein and may be incorporated onto the balloon 24 and/or stent 90 in any suitable fashion, including any of those disclosed herein.

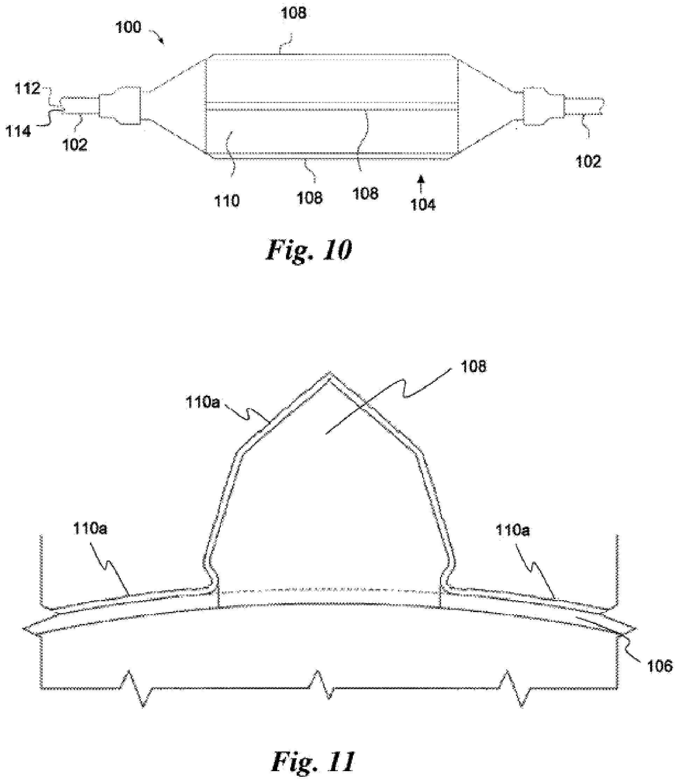

Figs. 10 and 11 depict another embodiment of the invention. Fig. 10 provides a side view of a drug-delivering scoring balloon according to one embodiment. Fig. 11 provides an enlarged cross-sectional view of a portion of the balloon of Fig. 10 including a dilatation element. More specifically, a drug-delivering scoring balloon catheter 100 includes a catheter shaft 102 and a balloon 104 mounted thereon. Balloon 104 has attached thereto, and preferably integrally formed with a balloon wall film 106 thereof, a plurality of dilation elements 108 projecting outwardly with respect to the balloon wall film 106 that spans between dilation elements 108. A material coat 110 including a layer containing drug and excipient 110a as described herein is carried by balloon 104 and in the specific illustrated embodiment by both the balloon wall film 106 and the dilation elements 108. Dilation elements 108 as depicted are trizoid-shaped elements; however, other shapes will be suitable for use in embodiments of the present invention, and dilation elements can be provided by separately attached or embedded articles or materials instead of being integrally formed with the balloon wall film. Catheter shaft 102 includes a first lumen 112 and second lumen 114. Lumen 112 is configured for inflation of balloon 104, and lumen 114 is configured to receive a guide wire or other guide member to be used in conjunction with balloon catheter 100. In the embodiment depicted, the layer containing drug and excipient 110a is directly adhered to outer wall surface of balloon 104 and in particular the outer surface of balloon wall film 106 and dilation elements 108. It will be understood that in other embodiments, this layer can be a part of a material coat that includes multiple layers, including any of those multiple layer coatings described hereinabove, and thus can have other coating layers underneath or overtop the layer containing drug and excipient. In addition or alternatively, the layer containing drug and excipient or material coat incorporating it can extend completely circumferentially around the balloon 104, coating both the dilation elements 108 and the balloon wall film 106 spanning between the dilation elements 108 (as in Figs. 10 and 11), or selective portions of the balloon 104 can be coated. Illustratively, the dilation elements 108 can be completely or partially coated with the layer containing drug and excipient or other material coat including it while the balloon wall film 106 spanning between the dilation elements 108 can be uncoated or at least free of the layer containing drug and excipient or other material coat including it; or, the balloon wall film 106 spanning between the dilation elements 108 can be completely or partially coated with the layer containing drug and excipient or other material coat including it while the dilation elements 108 can be uncoated or at least free of the layer containing drug and excipient or other material coat including it. These and other coating arrangements will be suitable herein. As well, while the balloon 104 is shown in its expanded condition, it will be understood that embodiments herein will include balloon 104 in a folded condition, including for example any of those folded conditions, and structural features provided thereby, described hereinabove.

### Methods of treatment

In certain aspects, a coated medical device as described herein, preferably comprising a stent and/or balloon catheter carrying the drug layer, can be configured to, and used to, treat any suitable body passage in a manner including release of the drug to the wall tissue of the body passage. The body passage may for example be a vein, artery, biliary duct, ureteral vessel, body passage or portion of the alimentary canal. A coated medical device as described herein may be used to treat a coronary artery, carotid artery, or a peripheral artery or vein, including as examples a renal artery or vein, iliac artery or vein, femoral artery or vein, popliteal artery or vein, subclavian artery or vein, intercranial artery or vein, aorta, vena cava, or others. In preferred embodiments, the coated medical devices will treat or prevent stenosis or restenosis in a body passage such as any of those identified herein, although treatment of other conditions is contemplated for other embodiments of the invention.

In certain embodiments, the coated medical device is configured to, and used to, treat a narrowing of a peripheral artery or vein. Examples of such arteries include, but are not limited to, the femoral artery, the superficial femoral artery (artery below the branch for the profunda femoris artery), the popliteal artery and the infrapopliteal artery. Examples of such veins include, but are not limited to, the common iliac vein, external iliac vein, femoral vein, the popliteal vein and the lesser/greater saphenous vein.

The following examples illustrate the present invention. The examples and embodiments described herein are for illustrative purposes only and modifications or changes in light thereof will be suggested to one skilled in the art without departing from the scope of the present invention.

### Example 1: Coating of Sirolimus and EGCG or Tannic Acid onto the surface of a balloon catheter

The surface of a nylon 12 balloon (Cook Advance 18LP 7 mm x 40 mm, Cook Medical Incorporated) is coated with a solid-state sirolimus-based coating. The sirolimus surface density is between 3 and 7 microgram/mm². The coating also contains either epigallocatechin gallate (EGCG) or tannic acid (TA) at a surface density of between 0.3 and 1.75 microgram/mm².

The coating is applied using a spraying method. Typical coating settlings are listed in Table 1. At least two sliding guns are utilized to spray the surface of the balloon at a 45 degree angle. The fluid pressure is between 4 and 8 PSI and the atomization pressure between 8 and 12 PSI. The balloons are coated in an environmentally controlled chamber set at a temperature between 70 F and 110 F, a humidity between 20 and 70 RPH, and an air flow rate between 80 and 300 CFM. The spraying solution contains Sirolimus and EGCG or Tannic Acid (TA) dissolved in ethanol and water at the following percentages by weight: Sirolimus = 4 ± 1%; EGCG or TA = 1 ± 1 %; Ethanol = 63 ± 1 %; Water = 32 ± 1 %.

### Example 2: Dose density of sirolimus on the surface of a balloon as a function of sirolimus concentration in the spray solution

Nylon 12 balloons are spray coated with a mixture of sirolimus and EGCG using the settings listed in Table 1. The concentrations of sirolimus in the spraying solution vary from 0.2% to 4% (w/w). The w/w ratio of sirolimus to EGCG to water is kept constant at 10:1:80. The total weight of the spraying solution is kept constant and the weight of ethanol is adjusted to accommodate for the increasing amount of drug.

**Table 1**

| **COATER SETTINGS** | | |
|---|---|---|
| ***Chamber Conditions*** | **% Humidity (RPH)** | 50% |
| | **Temperature (F)** | 90 F |
| | **Air Flow (CFM)** | 207 CFM |
| ***Gun Settings*** | **Gun Tip (i.e Fan or Cone?)** | Fan Tip |
| | **Fluid Pressure (PSI)** | 7PSI |
| | **Atomization P. (PSI)** | 10 PSI |
| | **Gun Speed (in/sec)** | 2.5 in/sec |
| | Fluid ON | 002sec |
| | Fluid TIME | 240sec |
| | Air OFF Delay | 10 sec |
| | Air OFF | 005 sec |
| | Gun Delay | 99 sec |
| | ROTVFY | 0.99 sec |
| | PURGE | 360sec |
| | STEP | 001 sec |
| | Rotate | 88 sec |
| | LQ ON | 90 sec |
| | LQ OFF | 99 sec |
| | MOVE | 90 sec |
| ***Automated Program*** | **N. Table Heights (TH)** | 10 |
| *Program 302* | **Starting TH (from home)** | 9.38 in |
| | **Ending TH (from home)** | 6.68 in |
| | **TH increments** | 0.3 in |
| | **N. Passes per TH** | 1 |
| | **Total N. of Passes** | 10 |
| | **Travel Distance (in)** | 28.5 in |
| ***Flow Rating*** | **Flow Rate (Gun 1)** | 5.0g/min (@ 5PSI Fluid P.) |
| | **Flow Rate (Gun 2)** | 5.0g/min (@ 5PSI Fluid P.) |
| | | |

| **SPRAYING SOLUTION** | | |
|---|---|---|
| ***Formulation*** | Drug (Rapa) Weight (g) | 2.5 |
| *Rapa:EGCG:Water = 4:1:32* | EGCG Weight (g) | 0.625 |
| | Ethanol Weight (g) | 39.625 |
| | Water Weight (g) | 20 |
| | Total Final Weight (g) | 62.75 |
| ***Fractions*** | **RAPA Fraction (w/w)** | **0.03984** |
| | **EGCG Fraction (w/w)** | **0.00996** |
| | **Water % (w/w)** | **0.31873** |
| ***Usage*** | Tot. Weight Sprayed (g) | 50 g |
| | | |

| **TARGET SUBSTRATE(S)** | | |
|---|---|---|
| ***Balloon*** | **Balloons Size** | Adv18 (4mm x 6cm) |
| | **State of Production** | Post Folding & Tipping |
| ***Uniformity Stack*** | **N of Surrogates** | 8 surrogates |
| | **Surrogate Length** | 10 mm (14GTW in MS_206) |

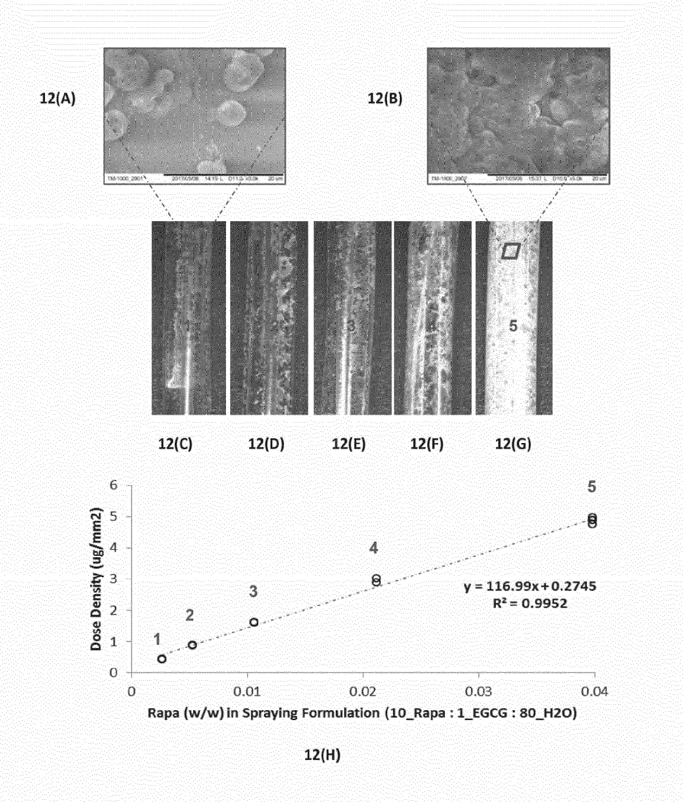

Figures 12(C-G) show digital light macroscopy images of the balloon surface at various sirolimus concentrations. Figures 12(A) and 12(B) are SEM images of the surfaces coated with sirolimus dose densities of 0.4 µg/mm² and 4.6 µg/mm². Figure 12(H) is a graph showing the dependency of sirolimus dose density on the surface as a function of sirolimus concentration in the spraying solution. The dose density is determined by HPLC.

### Example 3: Dose density of sirolimus on the surface of a balloon as a function of EGCG concentration in the spray solution

Nylon 12 balloons are spray coated with a mixture of sirolimus and EGCG, again using the settings listed in Table 1. The concentrations of ECGC in the spraying solution vary from 0.2% to 4% (w/w). The w/w ratio of sirolimus to EGCG to water vary from 10:1:80 (low EGCG) to 1:1:8 (High EGCG). The total weight of the spraying solution is kept constant and the weight of ethanol is adjusted to accommodate for the increasing amount of the excipient.

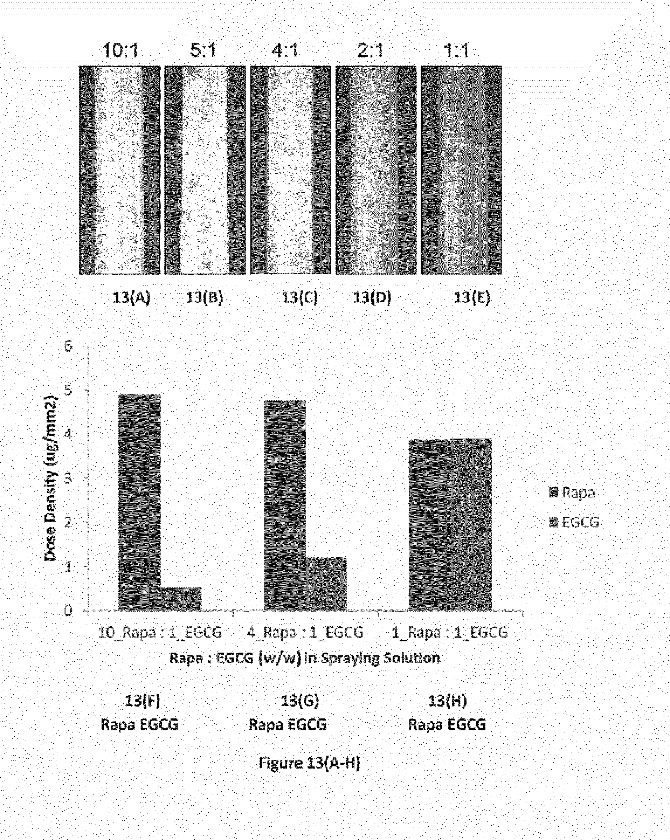

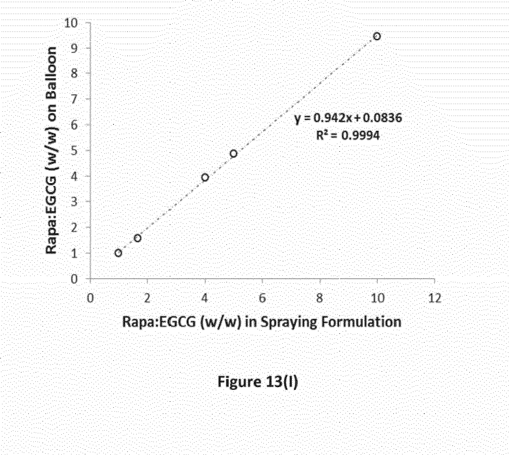

Figures 13(A-E) show digital light macroscopy images of the balloon surface at various EGCG concentrations. Figure 13(F-G) are bar charts showing the amounts of sirolimus and EGCG on the surface as a function of different ratios of sirolimus to excipient in the spraying solution. The surface density of drug and excipient on the surface of the balloon is determined via HPLC.

Figure 13(I) is a graph showing the ratio of drug to excipient as a function of their ratios in the straying solution.

### Example 4: Sirolimus particle size distribution on coated surface as a function of coating conditions.

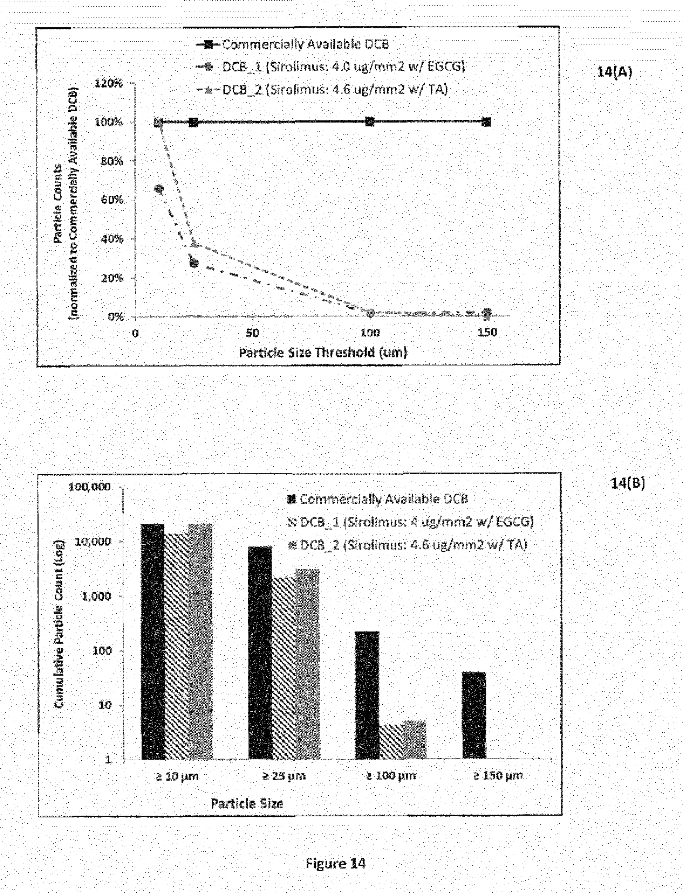

Nylon 12 balloons are spray coated with a mixture of sirolimus and EGCG or TA as disclosed above and analyzed for particulate release against a commercially available product. Briefly, drug coated balloons (DCBs) were inflated for 3 minutes at nominal pressure in clean water at 37C. Following inflation, balloons were deflated, and extracted. The water was then analyzed with a Liquid Particle counter. Figure 14 (A) is a graph showing average (n = 6) particulate counts of two sample balloons coated using the methods disclosed herein and normalized to a product that is commercially available in the U.S., Europe and possible other countries. Figure 14 (B) depicts the absolute data from the same experiment.

Although the invention has been described and illustrated with reference to specific illustrative embodiments thereof, it is not intended that the invention be limited to those illustrative embodiments. Those skilled in the art will recognize that variations and modifications can be made without departing from the true scope and spirit of the invention as defined by the claims that follow. It is therefore intended to include within the invention all such variations and modifications as fall within the scope of the appended claims and equivalents thereof.

### CLAUSES

1. A medical device comprising: a base structure having a surface, and a coating on the surface comprising a gallate excipient and particles of a Limus drug, wherein at least 70% of the particles having a maximum dimension greater than 10 micrometers have a maximum dimension less than 25 micrometers.
2. The medical device of clause 1, wherein at least 80% of the particles have a maximum dimension greater than 10 micrometers have a maximum dimension less than 25 micrometers.
3. The medical device of clause 1, wherein at least 20% of the particles have a maximum dimension greater than 10 micrometers have a maximum dimension between 25 micrometers and 100 micrometers.
4. The medical device of clause 1, wherein less than 0.5 % of the particles have a maximum dimension greater than 10 micrometers have a maximum dimension greater than 100 micrometers.
5. The medical device of clause 2, wherein less than 0.2 % of the particles having a maximum dimension greater than 10 micrometers have a maximum dimension greater than 100 micrometers.
6. The medical device of clause 1, wherein the excipient is selected from the group consisting of a gallate containing compound, epi gallo catechin gallate, tannic acid and epi catechin gallate.
7. The medical device of clause 6, wherein the excipient is tannic acid.
8. The medical device of clause 6, wherein the excipient is epi gallo catechin gallate.
9. The medical device of clause 1, wherein the medical device is selected from the group consisting of a stent, a vascular stent, a ureteral stent, a catheter, a balloon, a balloon catheter, a stent graft, a wire guide, and a cannula.
10. The medical device of clause 9, wherein the medical device is a balloon catheter.
11. The medical device of clause 1, wherein the Limus drug is present at a surface density of between 3 and 7 micrograms/mm² on a least a portion of the base surface.
12. The medical device of clause 1, wherein the Limus drug is selected from the group consisting of sirolimus, pimecrolimus, tacrolimus, everolimus, zotarolimus, novolimus, myolimus, temsirolimus, deforolimus and biolimus.
13. The medical device of clause 12, wherein the Limus drug is sirolimus.
14. The medical device of clause 1, wherein the coating is free of a polymer or non-polymer carrier matrix.
15. The medical device of clause 1, wherein the coating comprises less than 1 percentage by weight of a polymer or non-polymer carrier matrix.
16. The medical device of clause 1, wherein the excipient is present in an amount effective to increase a rate of release of the drug from the medical device when implanted in a patient.
17. A medical device comprising: a balloon having an outside surface, and a coating on the outside surface, the coating comprising particles of a crystalline form of sirolimus and an excipient, wherein at least 80% of the particles having a maximum dimension greater than 10 micrometers have a maximum dimension less than 25 micrometers, and wherein the excipient is selected from the group consisting of epi gallo catechin gallate and tannic acid.
18. A method for preparing a coating on a surface comprising: preparing a spraying solution comprising a solvent, a Limus drug and an excipient selected from the group consisting of epi gallo catechin gallate and tannic acid, wherein the solvent comprises water and a single organic solvent, and applying the spraying solution to the surface using a spray coating method.
19. The method of clause 18, wherein the solvent contains between 20% and 40% water.
20. The method of clause 18, wherein the single organic solvent is ethanol.
21. The method of clause 18, wherein the Limus drug is sirolimus.
22. The method of clause 18, wherein the excipient is tannic acid.
23. The method of clause 18, wherein the excipient is epi gallo catechin gallate.
24. The method of clause 18, wherein the solvent consists of water and a single organic solvent.
25. The method of clause 18, wherein the surface comprises a polymer.
26. The method of clause 18, wherein the applying is at a temperature between 70 F and 110 F.
27. The method of clause 18, wherein the applying is at a humidity between 20 and 70 RPH.
28. A medical device comprising: a base structure having a surface, and a coating on the surface comprising a gallate excipient and particles of a Limus drug, wherein when placed in an physiological environment at least 70% of the particles released from the coating having a maximum dimension greater than 10 micrometers have a maximum dimension less than 25 micrometers.
29. The medical device of clause 28, wherein at least 80% of the particles released having a maximum dimension greater than 10 micrometers have a maximum dimension less than 25 micrometers.
30. The medical device of clause 28, wherein at least 20% of the particles released having a maximum dimension greater than 10 micrometers have a maximum dimension between 25 micrometers and 100 micrometers.
31. The medical device of clause 28, wherein less than 0.5 % of the particles released having a maximum dimension greater than 10 micrometers have a maximum dimension greater than 100 micrometers.
32. The medical device of clause 28, wherein less than 0.2 % of the particles released having a maximum dimension greater than 10 micrometers have a maximum dimension greater than 100 micrometers.
33. An inflatable balloon comprising: a base structure having a surface, and a coating on the surface comprising a gallate excipient and particles of a Limus drug, wherein when placed in double distilled water at 37C and inflated for 3 minutes, at least 70% of the particles released from the coating having a maximum dimension greater than 10 micrometers have a maximum dimension less than 25 micrometers.
34. The inflatable balloon of clause 33, wherein at least 80% of the particles released having a maximum dimension greater than 10 micrometers have a maximum dimension less than 25 micrometers.
35. The inflatable balloon of clause 33, wherein at least 20% of the particles released having a maximum dimension greater than 10 micrometers have a maximum dimension between 25 micrometers and 100 micrometers.
36. The medical device of clause 33, wherein less than 0.5 % of the particles released having a maximum dimension greater than 10 micrometers have a maximum dimension greater than 100 micrometers.
37. The medical device of clause 33, wherein less than 0.2 % of the particles released having a maximum dimension greater than 10 micrometers have a maximum dimension greater than 100 micrometers.

## Claims

1. A medical device comprising:
a base structure having a surface, and
a coating on the surface comprising a gallate excipient and particles of a Limus drug, wherein at least 70% of the particles having a maximum dimension greater than 10 micrometers have a maximum dimension less than 25 micrometers.

2. The medical device of claim 1, wherein at least 80% of the particles having a maximum dimension greater than 10 micrometers have a maximum dimension less than 25 micrometers.

3. The medical device of claim 1 or claim 2, wherein at least 20% of the particles having a maximum dimension greater than 10 micrometers have a maximum dimension between 25 micrometers and 100 micrometers.

4. The medical device of any preceding claim, wherein less than 0.5 %, preferably less than 0.2%, of the particles having a maximum dimension greater than 10 micrometers have a maximum dimension greater than 100 micrometers.

5. The medical device of any preceding claim, wherein the excipient is selected from the group consisting of a gallate containing compound, epi gallo catechin gallate, tannic acid and epi catechin gallate, in particular wherein the excipient is tannic acid.

6. The medical device of any preceding claim, wherein the medical device is selected from the group consisting of a stent, a vascular stent, a ureteral stent, a catheter, a balloon, a balloon catheter, a stent graft, a wire guide, and a cannula, in particular, wherein the medical device is a balloon catheter.

7. The medical device of any preceding claim, wherein the Limus drug is present at a surface density of between 3 and 7 micrograms/mm² on a least a portion of the base surface.

8. The medical device of any preceding claim, wherein the Limus drug is selected from the group consisting of sirolimus, pimecrolimus, tacrolimus, everolimus, zotarolimus, novolimus, myolimus, temsirolimus, deforolimus and biolimus, preferably wherein the Limus drug is sirolimus.

9. The medical device of any preceding claim, wherein the coating comprises less than 1 percentage by weight of a polymer or non-polymer carrier matrix, in particular wherein the coating is free of a polymer or non-polymer carrier matrix.

10. A medical device comprising:
a balloon having an outside surface, and
a coating on the outside surface, the coating comprising particles of a crystalline form of sirolimus and an excipient, wherein at least 80% of the particles having a maximum dimension greater than 10 micrometers have a maximum dimension less than 25 micrometers, and wherein the excipient is selected from the group consisting of epi gallo catechin gallate and tannic acid.

11. A method for preparing a coating on a surface comprising:
preparing a spraying solution comprising a solvent, a Limus drug and an excipient selected from the group consisting of epi gallo catechin gallate and tannic acid, wherein the solvent comprises water and an organic solvent, and
applying the spraying solution to the surface using a spray coating method.

12. The method of claim 11, wherein the solvent contains between 20% and 40% water.

13. The method of claim 11 or claim 12, wherein the organic solvent is ethanol.

14. The method of any of claims 11-13, wherein the Limus drug is sirolimus.

15. The method of any of claims 11-14, wherein the solvent consists of water and one or more organic solvents, for example wherein the solvent consists of water and a single organic solvent.
